(19) Europäisches Patentamt
European Patent Office
Office européen des brevets

(11) Veröffentlichungsnummer: **0 203 509 B1**

# (12) EUROPÄISCHE PATENTSCHRIFT

(45) Veröffentlichungstag der Patentschrift: **03.04.91**

(51) Int. Cl.5: **C12Q 1/37**, //C12Q1/56, G01N33/86,A61K35/38, G01N33/68

(21) Anmeldenummer: **86106881.5**

(22) Anmeldetag: **21.05.86**

## (54) Verfahren zur quantitativen Bestimmung von Protein C und Aktivatorpräparat zur Durchführung des Verfahrens.

(30) Priorität: **29.05.85 CH 2267/85**
**25.09.85 CH 4135/85**
**28.11.85 CH 5087/85**

(43) Veröffentlichungstag der Anmeldung:
**03.12.86 Patentblatt 86/49**

(45) Bekanntmachung des Hinweises auf die Patenterteilung:
**03.04.91 Patentblatt 91/14**

(84) Benannte Vertragsstaaten:
**AT BE CH DE FR GB IT LI NL SE**

(56) Entgegenhaltungen:
**EP-A- 0 137 269** **GB-A- 1 293 795**
**US-A- 4 027 012** **US-A- 4 137 127**
**US-A- 4 341 762** **US-A- 4 485 176**

**THROMBOSIS & HAEMOSTASIS, Band 51, Nr. 1, 1984, Seiten 1-5, Stuttgart, De; R.M.Bertina et al.: "The use of a functional amd immunologic assay for plasma protein C in the study of the heterogeneity of congenital protein C deficiency".**

(73) Patentinhaber: **Pentapharm A.G.**
**Engelgasse 109**
**CH-4052 Basel(CH)**

(72) Erfinder: **Stocker, Kurt F.**
**Blumenrain 12**
**CH-4147 Aesch(CH)**
Erfinder: **Svendsen, Lars G.**
**Reichensteinerstrasse 15**
**CH-4153 Reinach(CH)**

(74) Vertreter: **Braun, André**
**Patentanwalt VSP Murtengasse 5**
**CH-4501 Basel(CH)**

**EP 0 203 509 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 104, Nr. 7, 17. Februar 1986, Seite 219, Spalten 1,2, Zusammenfassung Nr. 47533g, Columbus, Ohio, US; T. EXNER et al.: "Detection of specific proenzyme activators in snake venoms by a new immunoabsorbant-chromogenic substrate method".

CHEMICAL ABSTRACTS, Band 105, Nr. 7, 18. August 1986, Seite 275, Spalten 1,2, Zusammenfassung Nr. 56839j, Columbus, Ohio, Us; J.D. KLEIN et al.:"Purification of protein C activator from the venom of the southern copperhead snake (Agkistrodon contortrix contortrix)".

CHEMICAL ABSTRACTS, Band 105, Nr. 9, 1. September 1986, Seite 214, Spalte 2, Zusammenfassung Nr. 74085p, Columbus, Ohio, US; K. STOCKER et al.: "Protein C activators in snake venoms".

CLINICAL CHEMISTRY, Band 29, Nr. 2, Februar 1983, Seiten 225-236, Washington D.C., US; J. FAREED et al.: "Diagnostic efficacy of newer synthetic-substrates methods for assessing cogulation variables: A critical overview"

CHEMICAL ABSTRACTS, Band 97, Nr. 5, 2. August 1982, Seite 375, Zusammenfassung Nr. 36731f, Columbus, Ohio, US; R.A.MARLAR et al.: "Mechanism of action of human activated protein C, a thrombin-dependent anticoagulant enzyme".

CHEMICAL ABSTRACTS, Band 100, Nr. 19, 7. Mai 1984, Seite 216, Zusaammenfassung Nr. 152824t, Columbus, Ohio, US; N.SALA et al.: "A functional assay of protein C in human plasma".

CHEMICAL ABSTRACTS, Band 76, Nr. 16, 10. April 1972, Seite 100,101, Zusammenfassung Nr. 81949k, Columbus, Ohio, US; D. MUNJAL et al.: "Immunological and histochemical identity of esterases and other antigens in elapid venoms".

CHEMICAL ABSTRACTS, Band 105, Nr. 9, 1. September 1986, Seite 214, Spalte 2, Zusammenfassung Nr. 74085p, Columbus,Ohio, US; K.STOCKER et al.: "Protein C activators in snake venoms".

## Beschreibung

Die vorliegende Erfindung betrifft ein Verfahren zur quantitativen Bestimmung des Zymogens Protein C und ein Aktivatorpräparat für die Durchführung des Verfahrens.

Protein C ist ein im Blutplasma von Mensch und Säugetieren enthaltenes Zymogen des Haemostasesystems, welches durch einen Komplex von Thrombin und dem unlöslichen Gefässwandprotein Thrombomodulin durch begrenzte Proteolyse zur Serinproteinase Protein $C_a$ aktiviert wird. Protein $C_a$ bewirkt einerseits die hydrolytische Spaltung der Gerinnungsfaktoren V (Accelerin) und VIII (antihaemophiler Faktor A) und andererseits eine Aktivierung der Fibrinolyse. Die Wirkung von Protein C wird durch Protein S, Phospholipid und Calcium potenziert und durch einen spezifischen, im Plasma enthaltenen Inhibitor gehemmt.

Dank dieser Eigenschaften und Wirkungen nimmt Protein C eine zentrale Stellung in der Regulierung der Haemostase ein; es verhindert die Gerinnung im Bereich intakter Blutgefässe, ohne die Blutstillung am Ort einer Gefässverletzung zu beeinträchtigen.

Protein C ist ein Glycoprotein mit einem Molekulargewicht von ca. 6'000, welches in Abhängigkeit von Vitamin K in der Leber synthetisiert ward. Es besitzt in seinem Molekül mehrere $\gamma$-Carboxyglutaminsäurereste, die für die Bindung von Calcium und die Bildung des Enzym-Phospholipidkomplexes nötig sind. Antikoagulantientherapie mit Vitamin K-Antagonisten führen zur Synthese von Acarboxy-Protein C, welches zwar ebenfalls Enzymaktivität besitzt, durch Phospholipid und Calcium jedoch nicht potenziert werden kann.

Vererbter oder erworbener Mangel oder molekulare Missbildung von Protein C führen beim Menschen zu erhöhter Thromboseneigung.

Ausführliche Beschreibungen von Protein C finden sich bei KISIEL, W. and DAVIE, E.W. Protein C, Methods in Enzymology 80 , 320-332 (1981) und WITT, I. Protein C - Ein neuer Faktor der Haemostase. In: L. Roka und E. Spanuth (Eds.) Neue Aspekte in der Gerinnungsdiagnostik S.1-16 (1984) Stuttgart, New York: Schattauer Verlag.

Protein C besitzt antigene Eigenschaften, die seine quantitative Bestimmung nach einer immunologischen Technik möglich machen.

Nach der enzymgebundenen Immunadsorptionstechnik (ELISA, siehe I. WITT, loc. cit.) verläuft die Protein C-Bestimmung so, dass man den spezifischen, gegen Protein C gerichteten Antikörper durch Immunisierung von Kaninchen gewinnt, ihn an einen Träger aus Kunststoff bindet und die Probe mit dem antikörperbeladenen Träger in Kontakt bringt, wobei das Antigen "Protein C" vom Antikörper gebunden wird. Hierauf ward Antikörper, der mit Peroxidase gekoppelt wurde, im Ueberschuss zugegeben, der sich nun an die noch freien antigenen Determinanten des adsorbierten Protein C bindet. Nach Auswaschen des überschüssigen, markierten Antikörpers wird die durch Immunadsorption gebundene Peroxidaseaktivität mittels o-Phenylendiamin bestimmt. Die gebundene Peroxidaseaktivität ist proportional der Protein C-Konzentration in der Probe. Im Handel ist eine Testkombination zur Protein C-Bestimmung nach der ELISA-Technik erhältlich (ELISA-Protein C, Boehringer Mannheim, BRD).

Nach einer immunoelektrophoretischen Methode (R.M. BERTINA, Thrombosis & Haemostasis 48(I), 1-5 (1982)) wird der gegen Protein C gerichtete Antikörper in einer Agaroselösung aufgenommen, daraus eine Platte gegossen, die Probe auf die Antikörperplatte aufgetragen und diese während mehreren Stunden in einer geeigneten Apparatur unter Gleichstrom gesetzt. Aus den Gelplatten wird nun nicht präzipitiertes Protein ausgewaschen und die entstandenen, raketenförmigen Präzipitinzonen werden durch Färbung mit Amidoschwarz sichtbar gemacht. Die Länge der Präzipitinzonen ist proportional der Protein C-Konzentration der Probe. Fertige Antikörperplatten zur Protein C-Bestimmung sind im Handel erhältlich, so z.B. "Assera®-Plate Protein C", Diagnostica Stago, Asnières, Frankreich.

Protein C kann ferner auch nach einem radioimmunologischen Prinzip bestimmt werden, indem man den spezifischen Anti-Protein C-Antikörper mit einem radioaktiven Isotop wie z.B. $^{125}I$ markiert und seine Bindung an Protein C in der Probe radiologisch misst. Eine radioimmunologische Methode zur Protein C-Bestimmung wurde von K. IKEDA und J. STENFLO in Thrombosis Research 39 , 297-306 (1985) beschrieben.

Die genannten immunologischen Methoden zur Protein C-Bestimmung sind jedoch mit mehreren Nachteilen behaftet. Zunächst sind hochgereinigte Präparate von Protein C zur Herstellung des spezifischen Antikörpers erforderlich, denn eine Verunreinigung des Antigens durch Plasmaproteine würde zu einem Antikörper mit zu breitem Bindungsvermögen führen, der falsch hohe Protein C-Konzentrationen in der Probe vortäuscht. Immunologische Methoden erfordern ferner einen derart grossen Aufwand an Arbeit, Apparaten und Zeit, dass ihre praktische Anwendung nur in Frage kommt, wo der Zustand des Patienten einerseits diesen Aufwand rechtfertigt und andererseits die lange Wartezeit zulässt. Schliesslich ist die diagnostische Aussagekraft von immunologischen Protein C-Bestimmungen darum beschränkt, weil diese

Methoden neben aktivierbarem, enzymatisch funktionsfähigem Protein C auch dessen pathologische Formen sowie auch verbrauchtes, an Inhibitor gebundenes, inaktiviertes Enzym erfassen.

Die aufwendige Reinigung von Protein C sowie die Herstellung von Antikörperpräparaten entfallen und die Bestimmung wird spezifisch für funktionsfähiges Protein C, wenn es nicht an seinen antigenen Eigenschaften, sondern an seiner Enzymfunktion gemessen werden kann.

Protein C kann funktionell bestimmt werden, indem man das Zymogen aktiviert und die entstandene Enzymaktivität an einem natürlichen oder synthetischen Substrat misst.

Nach der Methode von KISIEL und DAVIE (KISIEL, W. and DAVIE, E.W. Protein C Methods in Enzymology 80 , 320-332 (1961)) wird Protein C in Chromatographie-Fraktionen, unter Verwendung der Kaolin-Cephalin-Gerinnungszeit von menschlichem Citratplasma als Indikatorreaktion bestimmt. In einer ersten Stufe wird die Protein C-haltige Probe durch 30-60 Minuten Inkubation mit Thrombin aktiviert und danach der Thrombinüberschuss durch Zusatz von Antithrombin III und Heparin neutralisiert. In einer zweiten Stufe wird nun ein Aliquot des Aktivierungsgemisches mit Normalplasma versetzt, das Gerinnungssystem durch Zusatz von Calciumchlorid, Cephalin und Kaolin aktiviert und die Zeit bis zur Gerinnung gemessen. Der durch aktiviertes Protein C bewirkte Abbau von Faktor V und VIII verursacht eine Verlängerung der Gerinnungszeit im Vergleich zu einem Kontrolltest ohne Protein C; diese Verlängerung ist proportional dem Protein C-Gehalt der Probe.

Diese Methode erfasst zwar spezifisch das funktionsfähige Protein C, aber sie kann nur für inhibitorfreie Protein C-Präparate angewendet werden, weil der im Plasma enthaltene Protein C-Inhibitor das Enzym rascher inaktiviert, als es durch Thrombinaktivierung gebildet wird.

Nach der Methode von FRANCIS und PATCH (R.B. FRANCIS and M.J. PATCH. A functional assay for protein C in human plasma. Thromb. Res. 32 , 605-613 (1983)) wird die Störung durch Protein C-Inhibitor ausgeschaltet, indem man die Probe bzw. das zu untersuchende Patientenplasma mit Bariumcitrat behandelt, um das Protein C zu adsorbieren, während der Inhibitor in Lösung bleibt. Aus dem abzentrifugierten Adsorbat wird das Protein C durch Behandlung mit Natriummorpholinoäthylsulfat eluiert, gewaschen und durch 60 Minuten Inkubation mit α-Thrombin teilweise aktiviert. Das Thrombin wird hierauf mit Antithrombin III und Heparin inaktiviert und hierauf das Heparin mit Protaminsulfat neutralisiert. Die Protein C-Aktivität in der auf diese Weise vorbereiteten Probe wird hierauf an menschlichem Normalplasma, durch Messung der Verlängerung der Kaolin-Cephalin-Gerinnungszeit bestimmt.

Aus einer Verdünnungsreihe von Normalplasma und den damit erzielten Gerinnungszeiten wird eine Eichkurve erstellt, aus der nun der Protein C-Gehalt im Patientenplasma in Prozent der Norm abgelesen werden kann.

Die besonderen Nachteile der Methode von FRANCIS und PATCH liegen in ihrer Umständlichkeit und in der langen Inkubationszeit mit Thrombin, die trotzdem nicht zur vollständigen Aktivierung der vorliegenden Protein C-Menge führt, denn dazu wären bei der angewandten Versuchsanordnung 4 Stunden Inkubation bei 37° C erforderlich.

Die Arbeit von BERTINA et al. (R.M. BERTINA, A.W. BROEKMANS, C. KROMMENHOEK-van ES and A. van WYJNGAARDEN: The use of a functional and immunological assay for plasma protein C in the study of the heterogeneity of congenital protein C deficiency. Thrombosis and Haemostasis 51 , 1-5 (1984)) beschreibt ein Verfahren zum Protein C-Bestimmung in Patientenplasma, das anstelle der komplexen Hemmung der Kaolin-Cephalin-Gerinnungszeit die direkte Spaltung eines chromogenen Substrates als Indikatorreaktion verwendet. Nach dieser Vorschrift wird Protein C aus dem Patientenplasma durch Adsorption an Aluminiumhydroxid und Eluierung mit Aethylendiamintetraessigsäure abgetrennt, 45 Minuten bei 37° C mit Thrombin aktiviert, Thrombin mit Antithrombin III und Heparin gehemmt und schliesslich das aktivierte Protein C durch Messung der p-Nitroanilinabspaltung aus dem synthetischen, chromogenen Substrat Pyroglutamyl-L-Prolyl-L-Arginin-p-nitroanilid bestimmt.

Die in allen beschriebenen Verfahren zur funktionellen Protein C-Bestimmung in Plasma angewandte Adsorptionsphase und die Aktivierung mit Thrombin wirken sich nachteilig auf die Bestimmungsgenauigkeit und auf die Bestimmungsgeschwindigkeit aus.

Adsorption und Elution sind nicht quantitativ verlaufende, temperatur- und zeitabhängige Vorgänge, deren Durchführung standardisiert werden muss und entsprechend qualifiziertes Personal erfordern. Ausserdem verändern die zur Eluierung verwendeten Stoffe die Elektrolytzusammensetzung des Testmediums und beeinflussen dadurch die Umsetzung des Substrates.

Thrombin aktiviert Protein C langsam und unvollständig und es muss nach erfolgter Aktivierung durch Antithrombin III und Heparin oder durch Hirudin gehemmt werden, damit es nicht selbst mit dem Substrat reagiert und bei Verwendung eines natürlichen Substrates falsch tiefe oder, bei Verwendung eines synthetischen Substrates, falsch hohe Protein C-Werte vortäuscht. Damit aber zugesetztes Antithrombin und Heparin nicht ihrerseits die Kaolin-Cephalin-Gerinnungszeit verlängern und falsch hohe Resultate

verursachen, muss Heparin durch Protaminsulfat neutralisiert werden, wenn Protein C nach einer Gerinnungsmethode bestimmt werden soll. Der Einsatz von Thrombin als Aktivator zur photometrischen Bestimmung von Protein C setzt ausserdem voraus, dass das zur Messung der Protein C-Aktivität verwendete chromogene Substrat nicht oder nur wenig durch Thrombin selber gespalten wird.

Bis anhin sind keine praktisch anwendbaren, besseren Alternativen zur Protein C-Aktivierung mit Thrombin bekannt. Zwar wird die Aktivierung mit Thrombin durch Thrombomodulin stark beschleunigt; doch steht dieses wasserunlösliche Protein bis heute nicht in anwendungsbereiter Form zur Verfügung.

Ein ebenfalls beschleunigender Zusatz von Calcium kann in Plasma nicht erfolgen, weil dadurch das Gerinnungssystem und damit andere Proteinasen als Protein C aktiviert würden, die dann ihrerseits mit dem natürlichen oder synthetischen Substrat reagieren würden.

Der aus dem Gift der Russell-Viper isolierte Faktor X-Aktivator übt nach KISIEL und DAVIE zwar durch seine Proteinaseaktivität ebenfalls eine aktivierende Wirkung auf Protein C aus; diese Aktivierung verläuft jedoch noch viel langsamer als die durch Thrombin und kann darum nicht zur Bestimmung von Protein C angewendet werden. Trypsin, das ebenfalls eine proteolytische Aktivierung von Protein C bewirkt, kann nicht verwendet werden, weil es als unspezifische Proteinase zahlreiche andere Plasma-Zymogene sowohl aktiviert als auch inaktiviert und ausserdem auch mit den verwendeten Substraten reagiert.

Proteinasen mit thrombin-artiger Substratspezifität, wie das das Fibrinopeptid A abspaltende Batroxobin aus Bothrops atrox-Gift oder Ancrod aus Agkistrodon rhodostoma-Gift, das Fibrinopeptid B abspaltende Enzym aus Agkistrodon contortrix-Gift, das thrombozytenaktivierende Enzym Thrombocytin aus B. atrox-Gift aktivieren Protein C nicht, und die durch Staphylocoagulase aus Prothrombin gebildete Thrombincoagulase oder das durch Ecarin aus Prothrombin gebildete Meizothrombin weisen ähnliche Eigenschaften wie Thrombin auf und bieten deshalb diesem gegenüber keine Vorteile.

Ueberraschenderweise wurde nun aber gefunden, dass ein Protein aus dem Gift der Kupferkopfschlange Agkistrodon contortrix, das keine thrombinartige Wirkung auf Fibrinogen und Thrombozyten ausübt, das im Gegensatz zu Thrombin weder Fibrinopeptid A noch B aus Fibrinogen abspaltet und weder Aggregations- noch Freisetzungsreaktionen an Thrombozyten auslöst, eine sehr starke und rasche Aktivierung von gereinigtem Protein C bewirkt. Es wurde ferner gefunden, dass dieses Schlangengiftprotein Protein C in so stark verdünntem Plasma zu aktivieren vermag, dass der vorliegende Protein C-Inhibitor praktisch nicht wirken kann oder dass ein Aktivierungsprodukt entsteht, welches durch den Plasma-Protein C-Inhibitor nicht gehemmt werden kann, sodass die Notwendigkeit einer umständlichen Trennung von Protein C und Inhibitor durch Adsorption entfällt. Es wurde ausserdem gefunden, dass das Protein C-aktivierende Schlangengiftprotein keine nachweisbare Proteinaseaktivität ausübt und darum weder ein natürliches noch ein synthetisches Substrat angreift. Im Gegensatz dazu, übt Thrombin eine spaltende Wirkung auf synthetische Substrate sowie eine gerinnende Wirkung auf natürliche Substrate aus, was zur Folge hat, dass die unerwünschte Wirkung des Thrombins durch Zugabe einer genau bemessenen Menge eines spezifischen Inhibitors aufgehoben werden muss. Es wurde schliesslich gefunden, dass man mit diesem Aktivator Protein C so rasch aktivieren kann, dass eine funktionelle Bestimmung von Protein C mit herkömmlichen automatischen Apparaten möglich wird.

Die vorliegende Erfindung betrifft nun ein Verfahren zur Bestimmung von Protein C, in einem dieses letztere enthaltenden Medium, welches Verfahren dadurch gekennzeichnet ist, dass man auf das genannte Medium Gift der Schlange Agkistrodon contortrix oder Gift einer Schlangenart, welches mit dem Gift von Agkistrodon contortrix eine immunologische Kreuzreaktion eingeht, oder ein aus den genannten Giften erhältliches, das Protein C aktivierendes Aktivatorpräparat so lange einwirken lässt, bis das Zymogen Protein C maximal zu einer Proteinase mit Protein $C_a$-Aktivität aktiviert ist und die Menge des gebildeten aktivierten Protein C durch photometrische Messung der Menge des farbigen oder fluoreszierenden Spaltproduktes, welches durch die katalytische hydrolytische Wirkung des aktivierten Protein C auf ein synthetisches chromogenes Substrat entsteht, oder durch Messung der durch aktiviertes Protein C bewirkten Verlängerung der Gerinnungszeit eines natürlichen Substrates bestimmt, oder dass man dem genannten Medium ein synthetisches chromogenes Substrat und das genannte Gift oder das genannte Aktivatorpräparat zusetzt, die hydrolytische Freisetzung des farbigen oder fluoreszierenden Spaltproduktes aus dem genannten Substrat photometrisch verfolgt und aus der beobachteten maximalen Geschwindigkeit der Substrathydrolyse den Gehalt des genannten Mediums an Protein C errechnet.

Die Spezifität der Protein C-Aktivierung in menschlichem Citratplasma wurde mit Hilfe spezifischer Proteinasesubstrate und -inhibitoren, sowie durch Messungen an Faktorenmangelplasmen verifiziert. Durch Inkubation von Plasma mit dem erfindungsgemässen Aktivatorpräparat werden keine Enzyme aktiviert, die das Plasmakallikreinsubstrat Bz-L-Pro-L-Phe-L-Arg-pNA, das Faktor Xa-Substrat $CH_3SO_2$-D-Leu-Gly-L-Arg-pNA oder das Plasminsubstrat Tos-Gly-L-Pro-L-Lys-pNA spalten. Gemessen an den chromogenen Substraten H-D-CHG-L-Pro-L-Arg-pNA und H-D-Pro-L-Pro-L-Arg-pNA, werden nach Aktivierung mit dem erfindungsgemässen Aktivatorpräparat in Normalplasma und in Plasma mit einem Mangel an den Faktoren VII, XI oder X amidolytische Aktivitäten der gleichen Grössenordnung gefunden, während durch das erfindungsgemässe Aktivatorpräparat in Protein C-freiem menschlichem Plasma keine die Protein C-Substrate 2AcOH-H-D-Pro-L-Pro-L-Arg-pNA und 2AcOH-H-D-Lys(Cbo)-L-Pro-L-Arg-pNA spaltende Aktivität erzeugt werden konnte. Gemessen am chromogenen Substrat H-D-Pro-L-Pro-L-Arg-pNA, wird die durch den erfindungsgemässen Aktivator aus Plasma generierte Protein C-Aktivität durch Zusatz des spezifischen Thrombin-Inhibitors Hirudin oder durch Zusatz des polyvalenten menschlichen Urin-Trypsininhibitors nicht gehemmt. Zusatz des polyvalenten Proteinaseinhibitors Aprotinin zu Plasma vor der Inkubation mit dem erfindungsgemässen Protein C-Aktivator verhindert die Spaltung von chromogenen Protein C-Substraten vollkommen. Zusatz von Aprotinin nach erfolgter Aktivierung und bereits in Gang befindlicher Substratspaltung hemmt die Reaktion sofort und vollständig. Dementsprechend wird auch gereinigtes, mittels insolubilisiertem Thrombin aktiviertes humanes Protein C durch Aprotinin vollständig gehemmt. Diese ergebnisse beweisen, dass der erfindungsgemässe Aktivator spezifisch Protein C aktiviert und dabei weder zur Bil dung von Thrombin, Plasmin, Faktor $X_a$, Plasmakallikrein noch zur Entstehung einer anderen die chromogenen Protein $C_a$ -Substrate spaltenden Enzymaktivität führt. Die Genauigkeit der Protein C-Bestimmung in Plasma mit Hilfe des erfindungsgemässen Aktivators und eines chromogenen Substrates konnte ferner durch Aufstockung des physiologischen Plasma-Protein C- Spiegels mit zugesetztem Protein C bewiesen werden. Der im aufgestockten Plasma gefundene Protein C-Gehalt entsprach der Summe von physiologischer und zugesetzter Protein C-Menge. Ausserdem konnte die Funktionsfähigkeit des erfindungsgemässen Protein C-Bestimmungsverfahrens durch Aktivitätsmessungen in Gemischen von Protein C-freiem und normalem menschlichem Plasma sowie durch Aktivitätsmessungen an normalem menschlichem Plasma mit steigenden Zusätzen an Anti-Protein C-Antikörper nachgewiesen werden.

Die Erfindung betrifft ferner ein aus Schlangengiften erhältliches Aktivatorpräparat, welches die Eigenschaft besitzt, Protein C von Mensch und Wirbeltieren, z.B. Schaf, Ziege, Rind, Pferd, Schwein, Kaninchen und Huhn, in aktiviertes Protein C umzuwandeln.

Als Ausgangsmaterial zur Herstellung von erfindungsgemässen Protein C-Aktivatorpräparaten eignen sich Gifte von solenoglyphen Schlangen (also solchen mit beweglichen, röhrenförmigen Giftzähnen), die der Familie der Vipern (Viperidae), insbesondere dem Tribus der Grubenottern (Crotalinae) und innerhalb diesem der Gattung der Dreieckskópfe (Agkistrodon) angehören. Insbesondere eignet sich das Gift der Art A. contortrix, dasjenige ihrer Unterarten wie z.B. A. contortrix contortrix, A. contortrix laticinctus, A. contortrix mokeson, A. contortrix phaeogaster, A. contortrix pictigaster, sowie Gift von Arten, die mit Gift von A. contortrix eine immunologische Kreuzreaktion eingehen, wie z.B. A. pisci vorus, dasjenige ihrer Unterarten wie z.B. A. piscivorus piscivorus, A. piscivorus conanti, A. piscivorus leucostoma, und Gift der Art A. bilineatus und ihrer Unterarten wie z.B. A. bilineatus bilineatus, A. bilineatus taylori und A. bilineatus russeolus.

Eine Uebersicht über die zoologische Klassierung der Schlangenfauna findet sich bei G. UNDERWOOD, Classification and distribution of venomous snakes in the world. In: C.Y. LEE (Ed.) Snake venoms S. 15-40, Berlin, Heidelberg, New York: Springer-Verlag (1979); Erläuterungen zur immunologischen Kreuzreaktion zwischen Giften verschiedener Schlangenarten und Antikörpern aus Serum immunisierter Säugetiere findet sich bei S.A. MINTON, Common antigens in snake venoms, loc. cit., S. 847-862.

Die Isolierung und Reinigung des Protein C-Aktivators aus Schlangengift kann anhand von bekannten Methoden zur Proteintrennung wie z.B. fraktionierte Aethanol- oder Ammonsulfatfällungen, Hoch- oder Niederdruckchromatographie an Molekularsieb- oder Ionenaustauschsystemen, Affinitätschromatographie, präparative Elektrophorese oder durch eine Kombination mehrerer der genannten Techniken erfolgen. Eine aktuelle Uebersicht über Proteintrennverfahren findet sich bei R. SCOPES, Protein purification, New York, Heidelberg, Berlin: Springer-Verlag (1982).

Das erfindungsgemässe Aktivatorpräparat kann beispielsweise durch Chromatographie des oben definierten Schlangengiftes, z.B. des A. contortrix-Giftes, an einem Anionenaustauscher mit für die Bindung von Proteinen geeigneter Porosität, z.B. quervernetztem Diäthylaminoäthyldextran (DEAE-Sephadex® A-50) oder Diäthylaminoäthylcellulose, und Eluierung mit Natriumphosphatpuffer bei neutralem pH und zunehmender Ionenstärke, Ent salzung der Protein C-aktivierenden Fraktionen durch Ultrafiltration und anschliessende Lyophilisierung hergestellt werden.

Das auf diese Weise hergestellte Aktivatorpräparat, in einer Konzentration von 2 µg pro ml an menschlichem Fibrinogen geprüft, bewirkte innerhalb 10 Minuten keine Gerinnung und, an einer nicht erhitzten Humanfibrinplatte geprüft, innerhalb 15 Stunden keine Fibrinolyse.

Gemessen am synthetischen chromogenen Substrat H-D-Pro-L-Pro-L-Arg-pNA, wird gereinigtes menschliches Protein C bei pH 8, Ionenstärke 0,15 und 37°C durch das Aktivatorpräparat in einer Konzentration von 2 µg Protein pro 1 ml Testgemisch innerhalb von höchstens 10 Minuten aktiviert.

Die Protein C-aktivierende Wirkung des Aktivatorpräparates wird durch 15-stündige Inkubation desselben mit 2,5 µMol Diisopropylfluorophosphat pro 1 ml bei pH 8 oder durch 15-stündige Inkubation mit 1 mg Iodoacetamid pro 1 ml bei pH 7 oder durch Zusatz von 0,05 µMol Aethylendiamintetraessigsäure-dinatriumsalz pro 1 ml nicht vermindert. Die Protein C-aktivierende Wirkung des Aktivatorpräparates wird ferner weder durch die Thrombininhibitoren Antithrombin III, Heparin und Hirudin noch durch den polyvalenten Proteinaseinhibitor Aprotinin gehemmt.

Das erfindungsgemässe Aktivatorpräparat kann auch erhalten werden durch Lösen des Schlangengiftes in einem wässrigen Medium, Entfernung von unerwünschten Giftbestandteilen aus der Lösung entweder durch fraktionierte Alkoholfällung, fraktionierte Salzfällung oder Hitzebehandlung bei saurem pH zwecks Zubereitung einer vorgereinigten Giftfraktion, Weiterreinigung der erhaltenen vorgereinigten Giftfraktion durch Chromatographie an einem Anionenaustauscher mit für die Bindung von Proteinen geeigneter Porosität, z.B. quervernetztem Diäthylaminoäthyldextran oder Diäthylaminoäthylcellulose, und Eluierung mit Natriumphosphatpuffer bei neutralem pH und bei zunehmender Ionenstärke, weitere Chromatographie an einem Kationenaustauscher, z.B. quervernetztem Carboxymethyldextran oder Carboxymethylcellulose und Eluierung mit einem Natriumacetatpuffer bei saurem pH, Einengung des Protein C-aktivierenden Eluates durch Ultrafiltration, Entsalzung und Endreinigung des Konzentrates durch Chromatographie an einem Molekularsiebgel, z.B. einem quervernetzten Dextrangel, unter Verwendung von verdünnter wässriger Essigsäure als Eluierungsmittel und anschliessende Lyophilisierung.

Das auf diese Weise hergestellte Aktivatorpräparat zeichnet sich dadurch aus, dass es in einer Konzentration von 0,1-0,5 µg pro ml wässrigem Reaktionsgemisch bei pH 6-8 und bei einer Temperatur von 20-40 °C das in 0,05 ml normalem menschlichem Citratplasma enthaltene Protein C innerhalb von höchstens 10 Minuten maximal aktiviert, dass es in einer Konzentration von 5 µg pro ml Testgemisch weder eine Gerinnung von menschlichem Fibrinogen innert 10 Minuten noch eine Lyse von menschlichem Fibrin innert 15 Stunden bewirkt, dass es Prothrombin und Gerinnungsfaktor X nicht aktiviert, dass es aus Protein C-freiem Plasma keine amidolytische Aktivität generiert, dass dessen Protein C aktivierende Wirkung durch 15-stündige Inkubation mit 2,5 µMol Diisopropylfluorophosphat pro 1 ml bei pH 8 oder durch Inkubation mit 1 mg Iodoacetamid pro 1 ml bei pH 7 oder durch Zusatz von 0,05 µMol Aethylendiamintetraessigsäure-dinatriumsalz pro 1 ml nicht vermindert wird, dass die Protein C aktivierende Wirkung weder durch Thrombininhibitoren wie Antithrombin III, Heparin und Hirudin noch durch den polyvalenten Proteinaseinhibitor Aprotinin gehemmt wird, dass es seine Protein C aktivierende Wirkung durch 10-minütiges Erhitzen auf 70 °C bei pH 3 bis 8 oder durch 24-stündige Lagerung bei 20-25 °C bei pH 2 bis 8 nicht einbüsst, dass es nach 1 Stunde bei pH 9 eine signifikante Aktivitätsabnahme aufweist, dass es nach Zusatz von 4% Natriumdodecylsulfat oder von 5 µMol Mangan-II-lactat pro 1 ml seine Protein C aktivierende Wirkung verliert, dass es durch 24-stündige Behandlung mit Dithiothreitol bei pH 7 nur wenig an Aktivität einbüsst, dass es durch polyvalentes, gegen amerikanische Grubenottern gerichtetes Antiserum neutralisiert wird, dass es nach Reduktion mit Dithiothreitol und anschliessender Alkylierung mit Iodoacetamid in der Polyacrylamidgelelektrophorese in Anwesenheit von Natriumdodecylsulfat und Färbung mit Coomassieblau eine einzige Zone mit einer relativen elektrophoretischen Mobilität aufweist, die einem Molekulargewicht von 39.000 ± 3.000 entspricht, dass es in der analytischen Ultrazentrifugation eine Sedimentationskonstante ($S20_W$) von 2,65 ± 3% aufweist, was einem Molekulargewicht von 36.800 ± 5% entspricht, dass es an einer kalibrierten Säule von quervernetztem Dextrangel (Sephadex® G-100) mit einem spezifischen Volumen ($K_{av}$) eluiert wird, welches einem Molekulargewicht von 37.000 entspricht, dass es einen durch isoelektrische Fokussierung bestimmten isoelektrischen Punkt von 3,0 ± 0,2 aufweist, dass dessen spezifische Absorption in einprozentiger, wässriger Lösung bei 280 nm und 1 cm Schichtdicke

$$(A^{280}_{1cm}\ 1\%)$$

13,5 ± 0,5 beträgt, dass es einen Gehalt an Kohlehydraten von 20 ± 3% aufweist, dass es in einer Konzentration von 1 µg/ml Testansatz, bei Inkubation mit einem chromogenen Substrat nach Patentanspruch 5 oder 6 bei pH 7-8,5 und 37 °C keine grössere Zunahme der bei 405 nm und 1 cm Schicht dicke gemessenen Absorption als 0,01 pro Minute verursacht, dass es nach intravenöser Verabreichung in einer Dosis von 80 U pro kg Körpergewicht an Kaninchen, die aktivierte Partialthromboplastinzeit im Plasma um mindestens das Doppelte des Ausgangswertes verlängert, dass es nach einer intravenösen Dosis von 80 U pro kg Körpergewicht keine akuten toxischen Symptome und keine Verhaltensstörungen an Kaninchen auslöst, dass dessen mehrmalige subcutane Verabreichung beim Kaninchen die Bildung von Antikörpern anregt und dass der im Serum der gegen das Aktivatorpräparat immunisierten Kaninchen enthaltene Antikörper mit dem Antigen einen durch Immunodiffusion nachweisbaren, präzipitierenden Komplex bildet.

Das Aktivatorpräparat ist auch aus Zuchten von genetisch einheitlichen (klonierten) Mikroorganismen wie z.B. Escherichia coli oder Saccharomyces cerevisiae, die durch Genmanipulation zur Biosynthese des Protein C-Aktivators befähigt wurden, erhältlich.

Die genetische Umprogrammierung der betreffenden Mikroorganismen kann nach an sich bekannten Methoden, durch Rekombination ihrer die Erbinformation-tragenden Desoxyribonucleinsäure (DNA) mit einer das Programm zur Biosynthese von Protein C-Aktivator-tragenden DNA-Kette (Gen) erzielt werden.

Zur Gewinnung von Genen, welche das Programm zur Biosynthese des Protein C-Aktivators tragen, kann entweder eine DNA-Kette nach dem Vorbild der Primärstruktur des Protein C-Aktivators derart synthetisiert werden, dass ihre Basenreihenfolge die Aminosäurereihenfolge des Aktivators bei der Biosynthese diktiert, es können ferner DNA-Ketten durch chemische Eingriffe so modifiziert werden, dass die erforderliche Basenreihenfolge entsteht oder es können natürliche Gene aus Zellen von Schlangenarten isoliert werden, welche das Programm zur Biosynthese des Protein C-Aktivators beinhalten.

Die Grundprinzipien der Gentechnologie wurden von E.L. WINNACKER, "Gene und Klone", Weinheim, VCA-Verlag (1985) beschrieben.

Das erfindungsgemässe Aktivatorpräparat ist auch befähigt, Protein C im lebenden Organismus von Wirbeltieren zu aktivieren. Injiziert man Kaninchen das Aktivatorpräparat intravenös und misst vor und nach der Injektion die aktivierte Partialthromboplastinzeit in Plasmaproben der Versuchstiere, so ist eine deutliche Verlängerung der Gerinnungszeit festzustellen. Diese Verlängerung der aktivierten Partialthromboplastinzeit ist auf eine Zerstörung der Faktoren $V_a$ und $VIII_a$ während des Gerinnungsvorganges zurückzuführen.

Keines der Tiere zeigte Zeichen toxischer Wirkungen des Aktivatorpräparates. Diese Ergebnisse zeigen, dass das erfindungsgemässe Aktivatorpräparat ausser zur Bestimmung von Protein C auch für pharmakologische Arbeiten über Protein C-Wirkungen an Versuchstieren eingesetzt werden kann. Dieses Aktivatorpräparat ist ferner als antithrombotisch wirksames Medikament in der Human- und Veterinärmedizin verwendbar.

Als natürliche Substrate zur Messung der Wirkung von aktiviertem Protein C über eine Inaktivierung der Faktoren V und VIII im Gerinnungstest kann man frisches, gefrorenes oder lyophilisiertes Blutplasma von Mensch oder Säugetieren mit den üblichen Calciumionen bindenden Zusätzen, wie Citrat oder Oxalat, oder Plasmazubereitungen, welche durch Erhitzung, pH-Umstellung oder Behandlung mit Enzymen, Adsorptions- oder Proteinfällungsmitteln von Inhibitoren oder von für eine Protein C-Bestimmung nicht relevanten Komponenten befreit worden sind, verwenden. Es können auch aus Blutplasma hergestellte Gerinnungsfaktorenkonzentrate oder deren Nebenprodukte, welche für therapeutische Zwecke Verwendung finden, und ferner Faktorenmangelplasmen verwendet werden.

Als synthetische Substrate für die direkte photometrische Messung der Aktivität des aktivierten Proteins C eignen sich Oligopeptide, insbesondere Di- oder Tripeptidyl-L-argininderivate, deren C-terminales Arginin über eine enzymatish durch aktiviertes Protein C spaltbare Amidbindung mit einer chromogenen Gruppe verbunden ist, und deren Salze mit Mineralsäuren oder organischen Säuren.

Es können insbesondere Verbindungen der Formel

$$R^2 - D - NH - \underset{\substack{|\\ (CH_2)_n \\ | \\ NH\ -\ R^3}}{CH} - \overset{\substack{O\\ \|}}{C} - L - Pro - L - Arg - R^1$$

in welcher n die Zahl 3 oder 4 bezeichnet, $R^2$ Wasserstoff oder

a) eine geradkettige oder verzweigte Alkanoylgruppe mit 2 bis 6 Kohlenstoffatomen,

b) eine ω-Carboxyl-, ω-Methoxycarbonyl- oder ω-Aethoxycarbonylalkanoylgruppe mit 2 bis 4 Kohlenstoffatomen im Alkanoyl,

c) eine geradkettige oder verzweigte Alkoxycarbonylgruppe mit 1 bis 4 Kohlenstoffatomen im Alkoxy,

d) eine Alkylsulfonylgruppe mit 1 bis 2 Kohlenstoffatomen im Alkyl,

e) eine unsubstituierte oder substituierte Benzoylgruppe oder

f) eine im Kern unsubstituierte oder substituierte Benzyloxycarbonylgruppe darstellt,

$R^3$ Wasserstoff oder eine gemäss a) bis und mit f) für $R^2$ definierte Gruppe und zudem, wenn n = 3 ist, eine Amidi no-oder Tosylamidinogruppe darstellt, und $R^1$ eine p-Nitrophenylamino-, 1- oder 2-Naphthylamino-, 4-Methoxy-2-naphthylamino-, 4-Methylcumaryl-(7)-amino-, 1,3-Di(methoxycarbonyl)-phenyl-(5)-amino-, Chinonylamino- oder Nitrochinonylaminogruppe darstellt, und deren Salze mit einer Mineralsäure oder organischen Säure verwendet werden.

Als Beispiele solcher synthetischer Substrate können H-D-Pro-L-Pro-L-Arg-pNA, D-Pyroglu-L-Pro-L-Arg-pNA, H-D-Lys(ε-Cbo)-L-Pro-L-Arg-pNA, H-D-Lys-L-Pro-L-Arg-pNA und deren Salze, insbesondere deren Hydrochloride und Acetate, genannt werden.

Da das erfindungsgemässe Protein C-aktivierende Aktivatorpräparat (Schlangengiftprotein) im Gegensatz zu Thrombin keinerlei nachweisbare Proteinaseaktivität ausübt und darum auch kein synthetisches Protein C-Substrat zu spalten vermag, können zur Bestimmung von aktiviertem Protein C nach dem erfindungsgemässen Verfahren auch solche Substrate verwendet werden, welche bei den herkömmlichen photometrischen Methoden zur Bestimmung von Protein C in Plasma unbrauchbar sind, weil sie nicht nur durch aktiviertes Protein C, sondern auch durch das zur Aktivierung eingesetzte Thrombin gespalten werden.

In diese Kategorie von synthetischen Substraten fallen beispielsweise die folgenden Verbindungen: 2AcOH.H-D-CHG-L-Pro-L-Arg-pNA, 2AcOH.H-D-CHG-L-Ala-L-Arg-pNA, Tos-Gly-L-Pro-L-Arg-pNA.AcOH und Phenylsulfonyl-Gly-L-Pro-L-Arg-pNA.AcOH.

Die in den obigen Formeln benützten Abkürzungen haben die folgende Bedeutung: Ala = Alanin; Arg = Arginin; Cbo = Carbobenzoxy; CHG = Cyclohexylglycin; Lys = Lysin; pNA = p-Nitroanilid; Pro = Prolin; Pyroglu = Pyroglutaminsäure.

Das erfindungsgemässe Verfahren erlaubt fer ner, die Aktivität von Protein C-Inhibitoren quantitativ zu bestimmen, indem man einer bekannten Menge Protein C die inhibitorhaltige Probe beimengt, hierauf das Protein C mit dem Aktivatorpräparat in aktiviertes Protein C überführt, nach einer angemessenen Reaktionszeit die nicht gehemmte Protein $C_a$-Aktivität mittels eines synthetischen oder natürlichen Substrates bestimmt und aus der Differenz zwischen vorgelegter und restlicher Protein $C_a$-Aktivität den Inhibitorgehalt errechnet.

## Beispiel 1

Herstellung eines Protein C-Aktivatorpräparates aus A. contortrix-Gift

200 mg Agkistrodon contortrix-Gift wurden in 1 ml 0,015 M Natriumphosphatpuffer, pH 6,8 gelöst, zentrifugiert und der Ueberstand auf eine mit dem selben Puffer äquilibrierte Säule mit DEAE-Sephadex® A-50 (quervernetztes Diäthylaminoäthyldextran) vom Format 2,6 x 90 cm aufgetragen. Hierauf wurde mit einem linearen Gradienten, gemischt aus 0,015 M Natriumphosphatpuffer, pH 6,8, und 0,4 M Natriumchlorid in 0,015 M Natriumphosphatpuffer, pH 6,8, eluiert und Fraktionen von je 20 ml gesammelt. Die Protein C-aktivierende Wirkung der einzelnen Fraktionen wurde dadurch ermittelt, dass man käufliches Bariumcitrat-Eluat aus Humanplasma, 1 mU per ml (Plasma Barium Citrate Eluate, Sigma-Chemie GmbH, München, BRD) mit der Probe während 15 Minuten bei 37°C inkubierte, 0,1 ml davon zu 0,1 ml menschlichem Normal-Plasma pipettierte, dieses Gemisch bei 37°C mit 0,1 ml Kephalin-Ellagsäurereagenz (Actin®, Dade, Aguada, Puerto Rico, USA) und 0,1 ml 0,025 M Calciumchlorid versetzte, sofort eine Stopuhr in Gang brachte und die Zeit bis zur Gerinnung bestimmte. Die Protein C-Aktivator enthaltenden Proben bewirkten eine Verlängerung der Gerinnungszeit von 34 Sekunden (Kontrolle ohne Eluat) auf 60 bis 90 Sekunden, je nach Aktivatorgehalt.

Die Protein C-aktivierende Aktivität war in den Fraktionen 40-45 enthalten (siehe beiliegende Figur). Die vereinigten aktiven Eluate aus 8 Chromatogra phiechargen wurden durch Ultrafiltration eingeengt, salzfrei gewaschen, in 0,1 M Glycin, pH 7,4, aufgenommen und lyophilisiert. Es wurden 830 mg Lyophilisat mit einem Proteingehalt von 16,5% erhalten.

5 µg des erhaltenen Aktivatorpräparates (0,825 µg Protein) bewirkten bei 37°C und pH 8,0, innert 7,5 Minuten die maximale Aktivierung von 40 mU gereinigtem menschlichem Protein C, gemessen am synthetischen, chromogenen Substrat 2AcOH.H-D-Pro-L-Pro-L-Arg-pNA.

## Beispiel 2

Photometrische Bestimmung von gereinigtem Protein C

Von einer Stammlösung von menschlichem Protein C, isoliert und gereinigt durch Bariumcitratadsorption und anschliessende Elution, Chromatographie an quervernetzter Diäthylaminoäthyl-Agarose, Chromatographie an Dextransulfat-Agarose und präparativer Polyacrylamidgel-Elektrophorese, mit einem Proteingehalt von 1,1 mg per ml, wurde mit 0,1 M Tris-HCl-Puffer, pH 8,0, eine Verdünnungsreihe erstellt.

Der Protein C-Gehalt dieser Verdünnungen wurde dadurch bestimmt, dass man in einer Photometerküvette 0,200 ml einer Lösung von 0,025 mg/ml des nach Beispiel 1 hergestellten Protein C-Aktivators mit

0,010 ml Protein C-Verdünnung versetzte, während 7,5 Minuten bei 37°C inkubierte, 1,390 ml Tris-Imidazolpuffer, pH 8,4, Ionenstärke 0,3, und 0,400 ml des chromogenen Substrates 2AcOH.H-D-Pro-L-Pro-L-Arg-pNA, 4 µMol per ml, zugab und die durch freigesetztes p-Nitroanilin bewirkte Absorptionszunahme (ΔA) bei 405 nm kontinuierlich registrierte.

Aus der Absorptionszunahme pro Zeiteinheit wurde nach der folgenden Gleichung der Protein C-Gehalt der Probe errechnet:

$$\frac{\Delta A/Min \cdot V}{v \cdot \varepsilon} = U/ml\ Probe$$

V = Testvolumen
v = Probevolumen
ε = Millimolarer Extinktionskoeffizient von-p-Nitroanilin
U = Internationale Enzymeinheit, Enzymmenge, welche unter Standardbedingungen 1 µMol Substrat pro Minute umsetzt

Die gemessene durch aktiviertes Protein C bewirkte Substratspaltung ist proportional dem Protein C-Gehalt der Probe (siehe Tabelle 1).

Tabelle 1 Photometrische Gehaltsbestimmung von gereinigtem Protein C

| µl Protein C Stammlösung | ΔA/Min | U Protein C per ml Stammlösung |
|---|---|---|
| 10 | 0,080 | 16,1 |
| 12 | 0,098 | 16,4 |
| 15 | 0,126 | 16,9 |
| 18 | 0,152 | 16,9 |
| 20 | 0,166 | 16,6 |

Beispiel 3

Photometrische Bestimmung von Protein C in Plasma

Der Protein C-Gehalt von menschlichem Citratplasma wurde dadurch bestimmt, dass man in einer Photometerküvette 0,200 ml Protein C-Aktivator (hergestellt nach Beispiel 1, 0,025 mg/ml) mit 0,050 ml Plasma versetzte, während 7,5 Minuten bei 37°C inkubierte, 1,550 ml Tris-Imidazolpuffer, pH 8,4, Ionenstärke 0,3, und 0,200 ml des chromogenen Substrates 2AcOH.H-D-CHG-L-Pro-L-Arg-pNA, 4 µMol/ml zugab und die durch freigesetztes p-Nitroanilin bewirkte Absorptionszunahme bei 405 nm kontinuierlich registrierte.

Nach der unter Beispiel 2 angeführten Formel wurde ein Protein C-Gehalt von 0,90 U per ml Plasma errechnet.

Beispiel 4

Bestimmung von Protein C in Plasma nach einer Gerinnungsmethode

0,1 ml Reagenz zur Bestimmung der aktivierten Partialthromboplastinzeit (Actin®, DADE, Aguada, Puerto Rico, USA), 0,1 ml Plasma und 0,1 ml des nach Beispiel 1 gewonnenen Aktivators (200 µg/ml) wurden während 60 Sekunden bei 37° C inkubiert, mit 0,1 ml Calciumchloridlösung 0,025 M versetzt und mit einer Stopuhr die Zeit bis zum Gerinnungseintritt bestimmt.

Als Modell für Protein C-defizitäres Plasma wurde Normalplasma mit verschiedenen Dosen eines käuflichen Anti-Protein C-Antikörperpräparates (Merz und Dade, Düdingen, CH) versetzt.

Die Gerinnung von Normalplasma wird durch die Aktivierung von Protein C um ein mehrfaches verlängert; Zusatz von Anti-Protein C führt zu einer dosisabhängigen Gerinnungszeitverkürzung (Tabelle 2).

Tabelle 2

| Plasma (ml) | Protein C-Aktivator (ml) | Anti-Protein C (µl) | Gerinnungszeit (Sekunden) |
|---|---|---|---|
| 0,1 | - | - | 35,5 |
| 0,1 | 0,1 | - | 140,5 |
| 0,1 | 0,1 | 2,5 | 108,0 |
| 0,1 | 0,1 | 5,0 | 79,5 |
| 0,1 | 0,1 | 10,0 | 51,5 |

Beispiel 5

100 mg des nach Beispiel 1 gewonnenen Aktivators wurden in 100 ml physiologischer Natriumchloridlösung gelöst, der pH-Wert mittels NaOH 1 N auf 7,4 gestellt und die Lösung durch ein Membranfilter mit einer Porengrösse von 0,22 µ steril filtriert.

Drei Kaninchen wurden 1 ml dieser Lösung per kg Körpergewicht intravenös injiziert, Es wurde vor und 30 Minuten nach der Injektion die aktivierte Partialthromboplastinzeit in Plasmaproben der Versuchstiere gemessen. Bei jedem Tier wurde eine starke Verlängerung der Gerinnungszeit beobachtet; keines der Tiere zeigte Symptome von toxischen Wirkungen.

Die Ergebnisse dieses Versuches sind in Tabelle 3 zusammengefasst.

Tabelle 3

| Tier Nr. | Aktivierte Partialthromboplastinzeit (Sek.) vor Injektion | 30 Min. nach Injektion |
|---|---|---|
| 1 | 12,5 | 125 |
| 2 | 24 | 150 |
| 3 | 19,5 | 60 |

Beispiel 6

Herstellung eines hochgereinigten Protein C-Aktivatorpräparates aus A. contortrix-Gift

1 g A. contortrix-Gift wurde in 100 ml Wasser gelöst, der pH-Wert dieser Lösung mit o-Phosphorsäure 1 N auf 3,0 gestellt und dies saure Giftlösung während 10 Minuten in einem Wasserbad bei 70 $\pm$ 2 °C gehalten, anschliessend auf 20 °C gekühlt, der pH-Wert mit Natronlauge 1 N auf 7,2 gestellt, die trübe Lösung zentrifugiert und der Ueberstand mit destilliertem Wasser auf ein Volumen von 100 ml verdünnt, um so eine vorgereinigte Giftfraktion zu erhalten.

Die vorgereinigte Giftfraktion wurde auf eine mit 0,015 M Natriumphosphatpuffer, pH 6,8, äquilibrierte Säule mit DEAE-Sephadex® A-50 vom Format 2,6 x 90 cm aufgetragen und mit einem linearen Gradienten, gemischt aus 0,015 M Natriumphosphatpuffer, pH 6,8, und 0,4 M Natriumchlorid in 0,015 M Natriumphosphatpuffer, pH 6,8, eluiert und Fraktionen von je 20 ml gesammelt. Die Protein C-aktivierende Wirkung der einzelnen Fraktionen wurde dadurch ermittelt, dass man 0,1 ml menschliches Citratplasma mit 0,1 ml Probe (1 : 350 in Wasser verdünnte Fraktion) und 0,1 ml Kephalin-Ellagsäurereagenz (Actin®) und 0,1 ml 0,025 M Calciumchloridlösung versetzte, sofort eine Stopuhr in Gang brachte und die Zeit bis zur Gerinnung bestimmte. Die Protein C-Aktivator enthaltenden Proben bewirkten eine Verlängerung der Gerinnungszeit von 34 Sekunden auf bis zu 200 Sekunden, je nach Aktivatorgehalt.

Die Protein C aktivierenden Fraktionen wurden vereinigt, durch Ultrafiltration auf 1/10 vom Eluatvolumen eingeengt, in 0,05 M Natriumacetatpuffer, pH 5,0, aufgenommen und auf 100 ml gestellt, auf eine mit 0.05 M Natriumacetatpuffer, pH 5,0, äquilibrierte Säule von CM-Sephadex® C-50 aufgetragen, und mit einem linearen Gradienten, gemischt aus 0,05 M Natriumacetatpuffer, pH 5,0, und 0,4 M Natriumchlorid in 0,05 M Natriumacetatpuffer, pH 5,0, eluiert und Fraktionen von je 20 ml gesammelt, welche nach der oben beschriebenen Methode auf Protein C-aktivierende Wirkung geprüft wurden.

Die Protein C aktivierenden Fraktionen wurden zusammen gemischt, durch Ultrafiltration auf 1/25 ihres Volumens eingeengt, mit 1% Essigsäure in dest. Wasser auf 25 ml gestellt und auf eine mit 1% Essigsäure in Wasser äquilibrierte Säule von Sephadex® G-100 aufgetragen, mit 1% Essigsäure eluiert und Fraktionen von je 20 ml gesammelt, die wiederum nach der eingangs beschriebenen Methode auf Protein C-aktivierende Wirkung geprüft wurden.

Die Protein C-aktivierenden Fraktionen wurden vereinigt und lyophilisiert. Es wurde ein salzfreies Aktivatorpräparat erhalten, welches in der Polyacrylamidgel-Elektrophorese eine einzige Zone aufwies und eine Protein C-aktivierende Aktivität von 35 U per mg aufwies.

Eine Einheit (U) Protein C-Aktivator ist die Menge, welche die in 1 ml normalem, menschlichem Citratplasma enthaltene Menge Protein C unter Standardbedingungen vollständig aktiviert.

Beispiel 7

Gewinnung von aktiviertem Protein C

25 mg Aktivatorpräparat nach Beispiel 6 wurden in 100 ml 0,1 molarem Natriumbicarbonatpuffer, pH 8,3, 0,5 molar an NaCl, gelöst. Der Lösung wurden 5 g CNBr-Sepharose 4B (AB Pharmacia, Uppsala, S), welche zuvor in 0,001 N Salzsäure gewaschen worden war, zugesetzt. Die Mischung wurde während 2 Stunden bei 20-25° C gerührt. Nach beendeter Reaktion wurde die Mischung durch eine Glasfilternutsche G3 filtriert und das abfiltrierte insolubilisierte Aktivatorpräparat fünfmal mit je 30 ml Natriumbicarbonatpuffer der obigen Zusammensetzung gewaschen. Zur Absättigung von allfällig vorhandenen reaktionsfähigen CNBr-Gruppen wurde das insolubilisierte Aktivatorpräparat während 2 Stunden mit 100 ml 0,5%igem Aethanolamin in Natriumbicarbonatpuffer der obigen Zusammensetzung gerührt, wiederum auf einer Glasfilternutsche gesammelt und noch dreimal mit je 300 ml Natriumbicarbonatpuffer gewaschen.

Eine Einheit Bariumsulfat-Eluat aus menschlichem Plasma (Sigma-Chemie GmbH, München, BRD), gelöst in 100 ml destilliertem Wasser wurde durch ein Membranfilter, Porengrösse 0,4 μ, filtriert. Diese Lösung, die einen mittels D-Lys(Cbo)-L-Pro-L-Arg-pNA bestimmten Gehalt von 0,9 U Protein C per ml aufwies, wurde mit dem insolubilisierten Aktivatorpräparat versetzt, während 2 Stunden bei Raumtemperatur gerührt und hierauf durch eine Glasfilternutsche filtriert. Das Filtrat wies einen Gehalt von 0,95 U aktiviertem Protein C auf.

**Ansprüche**

1. Aktivatorpräparat, welches die Eigenschaft besitzt, das Zymogen Protein C von Mensch und Wirbeltieren in eine Proteinase mit Protein $C_a$-Aktivität umzuwandeln und welches weder eine fibrinogenkoagulierende noch eine fibrinogenspaltende Aktivität aufweist, erhältlich aus Gift der Schlange Agkistrodon contortrix oder Gift einer Schlangenart, welches mit dem Gift von Agkistrodon contortrix eine immunologische Kreuzreaktion eingeht, durch
   (a) Chromatographie einer Lösung des Giftes an einem Anionenaustauscher mit für die Bindung von Proteinen geeigneter Porosität, z.B. quervernetztem Diäthylaminoäthyldextran oder Diäthylaminoäthylcellulose,
   (b) Eluierung mit Natriumphosphatpuffer bei neutralem pH und zunehmender Ionenstärke,
   (c) Entsalzung der aktiven Fraktionen durch Ultrafiltration und
   (d) anschliessende Lyophilisierung.

2. Aktivatorpräparat nach Patentanspruch 1, in hoch gereinigter Form, erhältlich durch
   (a) Lösen des Schlangengiftes in einem wässrigen Medium,
   (b) Entfernung von unerwünschten Giftbestandteilen aus der Lösung entweder durch fraktionierte Alkoholfällung, fraktionierte Salzfällung oder Hitzebehandlung bei saurem pH zwecks Zubereitung einer vorgereinigten Giftfraktion,
   (c) Weiterreinigung der erhaltenen vorgereinigten Giftfraktion durch Chromatographie an einem Anionenaustauscher mit für die Bindung von Proteinen geeigneter Porosität, z.B. quervernetztem Diäthylaminoäthyldextran oder Diäthylaminoäthylcellulose,
   (d) Eluierung mit Natriumphosphatpuffer bei neutralem pH und bei zunehmender Ionenstärke,
   (e) weitere Chromatographie an einem Kationenaustauscher, z.B. quervernetztem Carboxymethyldextran oder Carboxymethylcellulose,
   (f) Eluierung mit einem Natriumacetatpuffer bei saurem pH,
   (g) Einengung des Protein C-aktivierenden Eluates durch Ultrafiltration,
   (h) Entsalzung und Endreinigung des Konzentrates durch Chromatographie an einem Molekularsiebgel, z.B. einem quervernetzten Dextrangel, unter Verwendung von verdünnter wässriger Essigsäure als Eluierungsmittel und
   (i) anschliessende Lyophilisierung.

3. Aktivatorpräparat nach Patentanspruch 1, dadurch gekennzeichnet, dass es in einer Konzentration von 2 μg Protein pro ml Testgemisch, bei pH 8 und Ionenstärke 0,15 und bei 37° C das in 0,05 ml normalem menschlichem Citratplasma enthaltene Protein C innerhalb von höchstens 10 Minuten maximal aktiviert und dass es in einer Konzentration von 2 μg Protein pro ml Testgemisch weder eine Gerinnung von menschlichem Fibrinogen innert 10 Minuten noch eine Lyse von menschlichem Fibrin innert 15 Stunden bewirkt.

4. Aktivatorpräparat nach Patentanspruch 2, dadurch gekennzeichnet, dass es in einer Konzentration von

0,1-0,5 µg pro ml wässrigem Reaktionsgemisch bei pH 6-8 und bei einer Temperatur von 20-40 °C das in 0,05 ml normalem menschlichem Citratplasma enthaltene Protein C innerhalb von höchstens 10 Minuten maximal aktiviert und dass es in einer Konzentration von 5 µg pro ml Testgemisch weder eine Gerinnung von menschlichem Fibrinogen innert 10 Minuten noch eine Lyse von menschlichem Fibrin innert 15 Stunden bewirkt.

**5.** Verfahren zur quantitativen Bestimmung von Protein C in einem dieses letztere enthaltenden Medium, dadurch gekennzeichnet, dass man

(a) auf das genannte Medium ein Aktivatorpräparat gemäss Patentansprüchen 1-4 so lange einwirken lässt, bis das Zymogen Protein C maximal zu einer Proteinase mit Protein $C_a$-Aktivität aktiviert ist und die Menge des gebildeten aktivierten Proteins C bestimmt, indem man

(1) die Menge des farbigen oder fluoreszierenden Spaltproduktes, welches durch die katalytische hydrolytische Wirkung des aktivierten Proteins C auf ein synthetisches chromogenes Substrat entsteht, misst, wobei diese Menge der Menge des in der Testmischung enthaltenen Proteins C proportional ist, oder indem man

(2) die durch aktiviertes Protein C bewirkte Verlängerung der Gerinnungszeit eines natürlichen Substrates misst, wobei diese Verlängerung der Menge des in der Testmischung enthaltenen Proteins C proportional ist, oder dass man

(b) dem genannten Medium ein synthetisches chromogenes Substrat und das genannte Aktivatorpräparat zusetzt, die hydrolytische Freisetzung des farbigen oder fluoreszierenden Spaltproduktes aus dem genannten Substrat photometrisch verfolgt und aus der beobachteten maximalen Geschwindigkeit der Substrathydrolyse den Gehalt des genannten Mediums an Protein C errechnet.

**6.** Verfahren nach Patentanspruch 5, dadurch gekennzeichnet, dass man als Protein C enthaltendes Medium Blutplasma oder Fraktionen desselben, Lösungen von gereinigtem Protein C, Eluate von Protein C-Adsorbaten, Organextrakte oder Gewebekulturfiltrate und -extrakte verwendet.

**7.** Verfahren nach den Patentansprüchen 5 und 6, dadurch gekennzeichnet, dass man als synthetisches chromogenes Substrat ein Oligopeptid, an welches eine durch aktiviertes Protein C abspaltbare chromogene Gruppe durch eine Amidbindung geknüpft ist, verwendet.

**8.** Verfahren nach den Patentansprüchen 5 bis 7, dadurch gekennzeichnet, dass man als synthetisches Substrat eine Verbindung der Formel

$$R^2 - NH - \underset{\substack{|\\(CH_2)_n\\|\\NH - R^3}}{CH} - \overset{\substack{O\\\|}}{C} - L - Pro - L - Arg - R^1$$

in welcher n die Zahl 3 oder 4 bezeichnet,
$R^2$ Wasserstoff oder

(a) eine geradkettige oder verzweigte Alkanoylgruppe mit 2 bis 6 Kohlenstoffatomen,

(b) eine ω-Carboxyl-, ω-Methoxycarbonyl- oder ω-Äthoxycarbonylalkanoylgruppe mit 2 bis 4 Kohlenstoffatomen im Alkanoyl,

(c) eine geradkettige oder verzweigte Alkoxycarbonylgruppe mit 1 bis 4 Kohlenstoffatomen im Alkoxy,

(d) eine Alkylsulfonylgruppe mit 1 bis 2 Kohlenstoffatomen im Alkyl,

(e) eine unsubstituierte oder substituierte Benzoylgruppe oder

(f) eine im Kern unsubstituierte oder substituierte Benzyloxycarbonylgruppe darstellt, $R^3$ Wasserstoff oder eine gemäss (a) bis und mit (f) für $R^2$ definierte Gruppe und zudem, wenn $n = 3$ ist, eine Amidino- oder Tosylamidinogruppe darstellt, und $R^1$ eine p-Nitrophenylamino-, 1- oder 2-Naphthylamino-, 4-Methoxy-2-naphthylamino-, 4-Methylcumaryl-(7)-amino-, 1,3-Di(methoxycarbonyl)-phenyl-(5)-amino-, Chinonylamino- oder Nitrochinonylaminogruppe darstellt, oder ein Salz der oben definierten Verbindung mit einer Mineralsäure oder organischen Säure verwendet.

**9.** Verfahren nach den Patentansprüchen 5 bis 8, dadurch gekennzeichnet, dass man als synthetisches

Substrat ein wasserlösliches Salz, z.B. das Hydrochlorid oder Acetat von H-D-Pro-L-Pro-L-Arg-pNA, L-Pyroglu-L-Pro-L-Arg-pNA, H-D-Lys(ε-Cbo)-L-Pro-L-Arg-pNA oder H-D-Lys-L-Pro-L-Arg-pNA verwendet.

**10.** Verfahren nach den Patentansprüchen 5 bis 8, dadurch gekennzeichnet, dass man als synthetisches Substrat eine der nachfolgend aufgezählten Verbindungen verwendet: 2AcOH.H-D-CHG-L-Pro-L-Arg-pNA, 2AcOH.H-D-CHG-L-Ala-L-Arg-pNA, Tos-Gly-L-Pro-L-Arg-pNA.AcOH und Phenylsulfonyl-Gly-L-Pro-L-Arg-pNA.AcOH.

**11.** Verfahren nach den Patentansprüchen 5 und 6, dadurch gekennzeichnet, dass man als natürliches Substrat zur Messung der Wirkung von aktiviertem Protein C über eine Inaktivierung der Faktoren V und VIII im Gerinnungstest frisches, gefrorenes oder lyophilisiertes Plasma von Mensch oder Wirbeltieren mit den üblichen Calciumionen bindenden Zusätzen, wie Citrat, Oxalat oder Äthylendiamintetraacetat, oder eine Plasmazubereitung, welche durch Erhitzung, pH-Umstellung oder Behandlung mit Enzymen, Adsorptions- oder Proteinfällungsmitteln von Inhibitoren oder von für eine Protein C-Bestimmung nicht relevanten Komponenten befreit wurde, verwendet.

**12.** Verwendung des Aktivatorpräparates gemäss Patentansprüchen 1 und/oder 2 als antithrombotisch wirksame Komponente in Arzneimitteln, insbesondere in Antithrombotika.

**13.** Antithrombotisches Arzneimittel, dadurch gekennzeichnet, dass es als antithrombotisch wirksame Komponente das Aktivatorpräparat gemäss Patentansprüchen 1 und/oder 2 enthält.

**14.** Verfahren zur Gewinnung von aktiviertem Protein C aus Protein C-haltigen wässrigen Medien, dadurch gekennzeichnet, dass man das Aktivatorpräparat gemäss Patentansprüchen 1 und/oder 2 zwecks Insolubilisierung an einen wasserunlöslichen Träger, z.B. CNBr-Sepharose, Putrescinagarose oder Epsilonaminocaproylagarose, bindet, das insolubilisierte Aktivatorpräparat zur Aktivierung des Proteins C auf das Protein C-haltige wässrige Medium einwirken lässt, das insolubilisierte Aktivatorpräparat nach erfolgter Umwandlung von Protein C in aktiviertes Protein C aus dem wässrigen Medium entfernt und das aktivierte Protein C nach an sich bekannten Verfahren aus dem waessrigen Medium isoliert.

## Claims

**1.** Activator preparation which has the property of converting the zymogen protein C into a proteinase having a protein $C_a$-activity and which has neither a fibrinogen-coagulating nor a fibrinogen-splitting activity, obtainable from venom of the snake Agkistrodon contortrix or from venom of a snake species which undergoes an immunologic cross reaction with the venom of Agkistrodon contortrix by

(a) chromatography of a solution of the venom on an anion exchanger having a porosity adapted for binding proteins, e.g. cross-linked diethylaminoethyldextran or diethylaminoethylcellulose,
(b) elution with sodium phosphate buffer at neutral pH and increasing ionic strength,
(c) desalting the active fractions by ultrafiltration, and
(d) subsequent lyophilization.

**2.** Activator preparation according to claim 1, in highly purified form, obtainable by

(a) dissolution of the snake venom in an aqueous medium,
(b) removal of undesired venom components from the solution either by fractionated alcohol precipitation, fractionated salt precipitation or heat treatment at an acid pH in order to prepare a prepurified venom fraction,
(c) further purification of the obtained prepurified venom fraction by chromatography on an anion exchanger having a porosity adapted for binding proteins, e.g. cross-linked diethylaminoethyldextran or diethylaminoethylcellulose,
(d) elution with sodium phosphate buffer at neutral pH and increasing ionic strength,
(e) further chromatography on a cation exchanger, e.g. cross-linked carboxymethyldextran or carboxymethylcellulose,
(f) elution with a sodium acetate buffer at acid pH,
(g) concentration of the protein C-activating eluate by ultrafiltration,
(h) desalting and final purification of the concentrate by chromatography on a molecular sieve gel, e.g. a cross-linked dextran gel, using diluted aqueous acetic acid as the eluent, and

(i) subsequent lyophilization.

3. Activator preparation according to claim 1 which, in a concentration of 2 µg of protein per ml of test mixture, at pH 8, ionic strength 0.15 and 37° C, causes maximum activation of the protein C present in 0.05 ml of normal human citrated plasma within at most 10 minutes, and which, in a concentration of 2 µg of protein per ml of test mixture causes neither coagulation of human fibrinogen within 10 minutes nor lysis of human fibrin within 15 hours.

4. Activator preparation according to claim 2 which, in a concentration of 0.1-0.5 µg per ml of aqueous reaction mixture, at pH 6-8 and at a temperature of 20-40° C, causes maximum activation of the protein C present in 0.05 ml of normal human citrated plasma within at most 10 minutes, and which, in a concentration of 5 µg per ml of test mixture, causes neither coagulation of human fibrinogen within 10 minutes nor lysis of human fibrin within 15 hours.

5. Method for the quantitative determination of protein C in a medium containing same, which comprises
   (a) reacting the said medium with an activator preparation according to claims 1-4 for a period of time sufficient to cause maximum activation of the zymogen protein C to a proteinase having protein $C_a$-activity and determining the quantity of activated protein C thus formed by
      (1) measuring the quantity of coloured or fluorescent split product formed as a result of the catalytic hydrolytic action of activated protein C on a synthetic chromogenic substrate, the said quantity being proportional to the quantity of protein C present in the test mixture, or
      (2) by measuring the prolongation of the clotting time of a natural substrate, the said prolongation being proportional to the quantity of protein C present in the test mixture, or
   (b) adding to the said medium a synthetic chromogenic substrate and the said activator preparation, following photometrically the hydrolytic release of the coloured or fluorescent split product from the said substrate and calculating the protein C content in the said medium from the observed maximum velocity of the substrate hydrolysis.

6. Method according to claim 5 wherein blood plasma or fractions thereof, solutions of purified protein C, eluates of protein C-adsorbates, organ extracts, or filtrates and extracts of tissue cultures are used as the protein C-containing medium.

7. Method according to claims 5 and 6 which comprises using as a synthetic chromogenic substrate an oligopeptide to which a chromogenic group susceptible of being split off by activated protein C is attached through an amide bond.

8. Method according to claims 5 to 7 which comprises using as a synthetic substrate a compound having the formula

$$R^{2}-NH-\underset{\substack{|\\(CH_2)_n\\|\\NH-R^{3}}}{CH}-\overset{\substack{O\\\|}}{C}-L-Pro-L-Arg-R^{1}$$

wherein n represents integer 3 or 4,
$R^2$ represents hydrogen, or
   (a) a straight or branched alkanoyl group having 2 to 6 carbon atoms,
   (b) an ω-carboxyl, ω-methoxycarbonyl or ω-ethoxycarbonyl-alkanoyl group having 2 to 4 carbon atoms in the alkanoyl,
   (c) a straight or branched alkoxycarbonyl group having 1 to 4 carbon atoms in the alkoxy,
   (d) an alkylsulfonyl group having 1 to 2 carbon atoms in the alkyl,
   (e) an unsubstituted or substituted benzoyl group, or
   (f) a benzyloxycarbonyl group the nucleus of which is unsubstituted or substituted, $R^3$ represents hydrogen, or a group as defined for $R^2$ according to (a) to (f), and besides represents an amidino or tosylamidino group, if n = 3, and $R^1$ represents a p-nitrophenylamino, 1- or 2-naphthylamino, 4-methoxy-2-naphthylamino, 4-methylcoumaryl-(7)-amino, 1,3-di-(methoxycarbonyl)-phenyl-(5)-amino,

quinonylamino or nitroquinonylamino group, or a salt thereof with a mineral or organic acid.

9. Method according to claims 5 to 8 wherein a water-soluble salt, e.g. the hydrochloride or acetate of H-D-Pro-L-Pro-L-Arg-pNA, L-Pyroglu-L-Pro-L-Arg-pNA, H-D-Lys(ε-Cbo)-L-Pro-L-Arg-pNA or H-D-Lys-L-Pro-L-Arg-pNA is used as a synthetic substrate.

10. Method according to claims 5 to 8 wherein one of the compounds listed hereafter is used as a synthetic substrate: 2AcOH.H-D-CHG-L-Pro-L-Arg-pNA, 2AcOH.H-D-CHG-L-Ala-L-Arg-pNA, Tos-Gly-L-Pro-L-Arg-pNA.AcOH and phenylsulfonyl-Gly-L-Pro-L-Arg-pNA.AcOH.

11. Method according to claims 5 and 6 wherein fresh, frozen or lyophilized plasma from man or vertebrates containing the usual calcium ion-binding additives, such as citrate, oxalate or ethylenediaminetetraacetate, or a plasma preparation from which inhibitors or components irrelevant to the protein C determination have been removed by heating, pH adjustment or treatment with enzymes, adsorbing or protein-precipitating agents, is used as a natural substrate for measuring the action of activated protein C via inactivation of factors V and VIII in the clotting test.

12. Use of the activator preparation according to claims 1 and/or 2, as an antithrombotically active component in drugs, particularly in antithrombotics.

13. Antithrombotic which contains, as an antithrombotically active component, the activator preparation according to claims 1 and/or 2.

14. Method for obtaining activated protein C from protein C-containing aqueous media, which comprises binding the activator preparation according to claims 1 and/or 2 to a water-insoluble carrier, e.g. CNBr-sepharose, putrescinagarose or epsilonaminocaproylagarose, for the purpose of insolubilization, reacting the insolubilized activator preparation with the protein C-containing aqueous medium for activating the protein C, removing the insolubilized activator preparation from the aqueous medium after completed conversion of protein C into activated protein C and isolating the activated protein C from the aqueous medium according to known methods.

## Revendications

1. Préparation activante, laquelle possède la propriété de transformer le zymogène protéine C en une protéinase présentant l'activité de la protéine $C_a$ et laquelle n'exerce ni une activité coagulante ni une activité décomposante sur le fibrinogène, ladite préparation pouvant être obtenue à partir du venin du serpent Agkistrodon contortrix ou à partir du venin d'une autre espèce de serpent, lequel venin entre en réaction croisée immunologique ("immunological cross reaction") avec le venin d'Agkistrodon contortrix, par
   (a) chromatographie d'une solution dudit venin sur un échangeur d'anions ayant une porosité adaptée à la rétention de protéines, tel que le diéthylaminoéthyldextran réticulé ou la diéthylaminoéthylcellulose,
   (b) élution avec un tampon phosphate de sodium à pH neutre et force ionique croissante,
   (c) déminéralisation des fractions actives par ultrafiltration et
   (d) lyophilisation subséquente.

2. Préparation activante selon la revendication 1, sous forme hautement purifiée, pouvant être obtenue par
   (a) dissolution du venin de serpent dans un milieu aqueux,
   (b) élimination, de la solution, des composantes indésirées du venin par précipitation alcoolique fractionnée, précipitation saline fractionnée ou traitement à chaud à pH acide dans le but de préparer une fraction de venin prépurifiée,
   (c) purification ultérieure de la fraction de venin prépurifiée obtenue, par chromatographie sur un échangeur d'anions ayant une porosité adaptée à la rétention de protéines, tel que le diéthylaminoéthyldextran réticulé ou la diéthylaminoéthylcellulose,
   (d) élution avec un tampon phosphate de sodium à pH neutre et force ionique croissante,
   (e) chromatographie ultérieure sur un échangeur de cations, tel que le carboxyméthyldextran réticulé ou la carboxyméthylcellulose,

(f) élution avec un tampon acétate de sodium à pH acide,
(g) concentration de l'éluat activant la protéine C par ultrafiltration,
(h) déminéralisation et purification finale du concentré par chromatographie sur un gel de séparation moléculaire, un gel de dextran réticulé par exemple, en utilisant de l'acide acétique aqueux dilué en tant qu'éluant et
(i) lyophilisation subséquente.

3. Préparation activante selon la revendication 1, caractérisée en ce qu'à une concentration de 2 $\mu$g de protéine par ml de mélange réactionnel, à pH 8, force ionique 0,15 et 37° C, elle active de façon maximale la protéine C présente dans 0,05 ml de plasma humain normal citraté en l'espace de 10 minutes au plus, et en ce qu'à une concentration de 2 $\mu$g de protéine par ml de mélange réactionnel, elle ne provoque ni une coagulation de fibrinogène humain en l'espace de 10 minutes ni une lyse de fibrine humaine en l'espace de 15 heures.

4. Préparation activante selon la revendication 2, caractérisée en ce qu'à une concentration de 0,1 à 0,5 $\mu$g par ml de mélange réactionnel aqueux, à pH 6-8 et à une température de 20-40° C, elle active de façon maximale la protéine C présente dans 0,05 ml de plasma humain normal citraté en l'espace de 10 minutes au plus, et en ce qu'à une concentration de 5 $\mu$g par ml de mélange réactionnel elle ne provoque ni une coagulation de fibrinogène humain en l'espace de 10 minutes ni une lyse de fibrine humaine en l'espace de 15 heures.

5. Méthode de dosage quantitatif de protéine C dans un milieu contenant ladite protéine, caractérisée en ce que
(a) on laisse agir une préparation activante selon les revendications 1-4 sur ledit milieu jusqu'à ce que le zymogène protéine C soit activé de façon maximale en une proteinase présentant l'activité de la protéine $C_a$ et on mesure la quantité de protéine C activée formée en mesurant
(1) la quantité de produit de scission coloré ou fluorescent engendrée par l'action catalytique hydrolytique de la protéine C activée sur un substrat chromogène synthétique, cette quantité étant proportionnelle à celle de la protéine C présente dans le mélange réactionnel, ou
(2) la prolongation, due à l'action de la protéine C activée, du temps de coagulation d'un substrat naturel, cette prolongation étant proportionnelle à la quantité de protéine C présente dans le mélange réactionnel, ou en ce que
(b) on ajoute audit milieu un substrat chromogène synthétique et ladite préparation activante, on suit par photométrie le dégagement hydrolytique du produit de scission coloré ou fluorescent à partir dudit substrat et on calcule la teneur en protéine C dudit milieu à partir de la vitesse maximale d'hydrolyse du substrat observée.

6. Méthode selon la revendication 5, caractérisée en ce que l'on utilise, en tant que milieu contenant la protéine C, du plasma sanguin ou ses fractions, des solutions de protéine C purifiée, des éluats de produits d'adsorption de protéine C, des extraits organiques ou des filtrats et extraits de cultures tissulaires.

7. Méthode selon les revendications 5 et 6, caractérisée en ce que l'on utilise en tant que substrat chromogène synthétique un oligopeptide auquel est lié par une liaison amidique un groupe chromogène susceptible d'être scindé par la protéine C activée.

8. Méthode selon les revendications 5 à 7, caractérisée en ce que l'on utilise, en tant que substrat synthétique un composé de formule

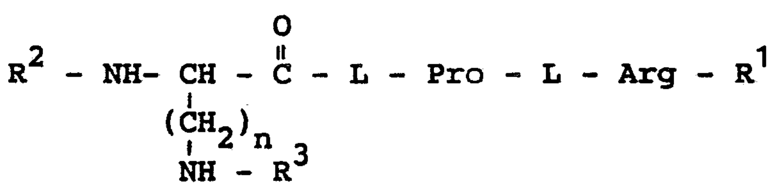

dans laquelle n représente le chiffre 3 ou 4,
$R^2$ représente l'hydrogène, ou

(a) un groupe alkanoyle à chaîne droite ou ramifiée contenant 2 à 6 atomes de carbone,

(b) un groupe ω-carboxyl-, ω-méthoxycarbonyl- ou ω-éthoxycarbonyl-alkanoyle contenant 2 à 4 atomes de carbone dans l'alkanoyle,

(c) un groupe alkoxycarbonyle à chaîne droite ou ramifiée contenant 1 à 4 atomes de carbone dans l'alkoxy,

(d) un groupe alkylsulfonyle contenant 1 à 2 atomes de carbone dans l'alkyle,

(e) un groupe benzoyle substitué ou non-substitué, ou

(f) un groupe benzyloxycarbonyle substitué ou non-substitué dans le noyau, $R^3$ représente l'hydrogène ou l'un des groupes selon (a) à (f) définis pour $R^2$ et en outre, lorsque n = 3, un groupe amidino ou tosylamidino, et $R^1$ représente un groupe p-nitrophénylamino, 1- ou 2-naphtylamino, 4-méthoxy-2-naphtylamino, 4-méthylcoumaryl-(7)-amino, 1,3-di(méthoxycarbonyl)-phényl-(5)-amino, quinonylamino ou nitroquinonylamino, ou un sel du composé défini ci-dessus avec un acide minéral ou organique.

**9.** Méthode selon les revendications 5 à 8, caractérisée en ce que l'on utilise en tant que substrat synthétique un sel soluble dans l'eau, par exemple l'hydrochlorure ou l'acétate de H-D-Pro-L-Pro-L-Arg-pNA, L-Pyroglu-L-Pro-L-Arg-pNA, H-D-Lys(ε-Cbo)-L-Pro-L-Arg-pNA ou H-D-Lys-L-Pro-L-Arg-pNA.

**10.** Méthode selon les revendications 5 à 8, caractérisée en ce que l'on utilise en tant que substrat synthétique l'un des composés ci-après: 2AcOH.H-D-CHG-L-Pro-L-Arg-pNA, 2AcOH.H-D-CHG-L-Ala-L-Arg-pNA, Tos-Gly-L-Pro-L-Arg-pNA.AcOH et phénylsulfonyl-Gly-L-Pro-L-Arg-pNA.AcOH.

**11.** Méthode selon les revendications 5 et 6, caractérisée en ce que l'on utilise en tant que substrat naturel pour mesurer, dans le test de coagulation, l'action de la protéine C activée, cette action comportant l'inactivation des facteurs V et VIII, du plasma frais, congelé ou lyophilisé d'origine humaine ou de vertébrés avec les additifs liant les ions calcium usuels, tels que le citrate, l'oxalate ou l'éthylènediaminetétraacétate, ou une préparation de plasma, de laquelle ont été éliminés les inhibiteurs ou les composantes n'étant pas significatives pour un dosage de protéine C par chauffage, ajustement de pH ou traitement par des enzymes, des agents adsorbants ou des agents précipitant les enzymes.

**12.** Utilisation de la préparation activante selon les revendications 1 et/ou 2 en tant que composant antithrombotique actif dans les médicaments, en particulier dans les antithrombotiques.

**13.** Médicament antithrombotique, caractérisé en ce qu'il contient la préparation activante selon les revendications 1 et/ou 2 en tant que composant antithrombotique actif.

**14.** Procédé d'obtention de protéine C activée à partir de milieux aqueux contenant de la protéine C, caractérisé en ce que l'on lie la préparation activante selon les revendications 1 et/ou 2 à un véhiculeur insoluble dans l'eau, par exemple le CNBr-sépharose, la putrescine agarose ou l'epsilonaminocaproylagarose, dans le but de la rendre insoluble, que l'on fait agir la préparation activante insolubilisée sur le milieu aqueux contenant la protéine C pour activer cette dernière, que l'on élimine du milieu aqueux la préparation activante insolubilisée après l'achèvement de la transformation de protéine C en protéine C activée et que l'on isole la protéine C activée du milieu aqueux selon des méthodes connues.

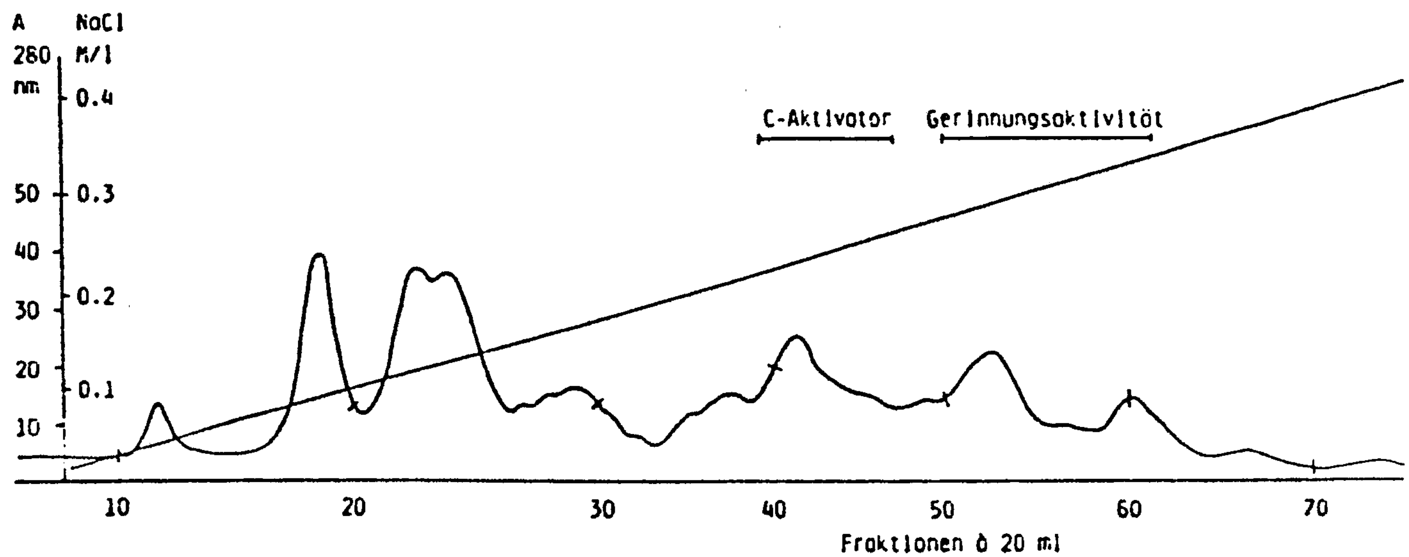


Fig. 1:

Isolierung von Protein C-Aktivator aus A. contortrix-Gift mittels Chromatographie an DEAE-Sephadex® A-50.